# EUROPEAN PATENT SPECIFICATION

(11) **EP 4 302 729 B1**
(45) Date of publication and mention of the grant of the patent: **18.02.2026**
(21) Application number: 21929282.8
(22) Date of filing: 05.04.2021
(51) Int. Cl.: A61F 2/82, A61F 2/00, A61F 2/07, B05C 13/02, B25B 11/00

(54) **VARIABLE COATING JIG FOR STENT HAVING SURFACE ROUGHNESS, AND STENT PRODUCED BY MEANS OF SAME**
VARIABLE BESCHICHTUNGSVORRICHTUNG FÜR EINEN STENT MIT OBERFLÄCHENRAUHEIT UND DAMIT HERGESTELLTER STENT
GABARIT DE REVÊTEMENT VARIABLE POUR STENT AYANT UNE RUGOSITÉ DE SURFACE, ET STENT PRODUIT AU MOYEN DE CELUI-CI

(30) Priority: 03.03.2021 KR 20210028385
(43) Date of publication of application: 10.01.2024
(73) Proprietor: SEWOON MEDICAL CO., LTD., Chungcheongnam-do, 31061 (KR)
(72) Inventor: LEE, Jae Hee, Seoul 02583 (KR); MOON, Soo Jung, Antioch,, California 94531 (US)
(74) Representative: Frenkel, Matthias Alexander
(86) International application number: PCT/KR2021/004176
(87) International publication number: WO 2022/186420

(56) References cited:
- KR-A- 20130 029 863
- KR-A- 20160 138 263
- KR-A- 20160 142 481
- KR-A- 20160 142 481
- KR-A- 20190 011 452
- KR-B1- 101 666 944
- US-A1- 2007 003 688
- US-A1- 2009 035 449

## Description

### TECHNICAL FIELD

The present invention relates to a coating jig for stents. In particular, the present invention relates to a variable coating jig for stents, having a surface roughness, which is used to form a thin membrane in a stent by applying a coating material.

### BACKGROUND

A stent is a medical device that is widely used to prevent stenosis of an organ, a blood vessel, and a biliary tract in humans, and the like. The stent forcibly enlarges a site where the stent is inserted to enable smooth flow of a transfer substance, such as a fluid like a bodily fluid, blood, gas, food, enzyme, etc.) in the organ. A thin film, i.e., a membrane, is formed on the stent to block tumors and cancer cells from infiltrating from an outside of the stent. Here, the membrane of the stent is formed in a coating process using a coating jig. Stents are made into different shapes and sizes depending on where the stents are used in a human body. This necessitates multiple coating jigs, which has given rise to an extremely high process cost and installation space.

Moreover, an existing membrane has a stickiness property due to material characteristics thereof. When the stent inserted into an insertion instrument, such as a catheter, is used, the stent is deployed at a lesion site. Thus, the stent is stored in the insertion instrument. During this time, an external force is exerted on the stent, and a diameter of the stent is minimized. Consequently, the stent elongates and is stored for a long period of time with the membrane contacting itself.

As such, the inner face of the membrane partially sticks to itself, so the membrane fails to return to its original shape when deployed from the insertion instrument due to its material characteristics, external environment, and the like. As a solution, an existing manufacturing process of the stent has added a process where costly silicon powder, oil or the like, certified with biocompatibility, is treated in the membrane during a production process of the stent. However, it has been found that the added process cannot effectively resolve the issue caused by the stickiness property of the membrane. US 2007/003688 A1 discloses a stent fixture for supporting a stent during formation of a coating. In the document the stent fixture is described to comprise a structure having arm elements extending from the structure, wherein the arm elements are configured to allow an inner side of a stent to rest on and be supported by the arm elements, a second structure for support an opposing end of the stent, wherein the second structure comprises arm elements extending out from the second structure, wherein the arm elements of the second structure are configured to allow the inner side of the stent to rest on and be supported by the arm elements of the second structure, and a third structure connecting the structure to the second structure and extending through a longitudinal bore of the stent.

### SUMMARY

Embodiments of the present invention have been made in an effort to solve the above-mentioned problems. An objective of the present invention is to provide a variable coating jig for stents, which is widely usable in stents having different shapes and sizes. From the above, a high process cost resulted from an existing coating process can be reduced, and an installation space designated for a coating jig may be minimized.

In addition, according to the present invention, provided is a coating jig for stents, having a surface roughness, which can decrease stickiness of a membrane without adding a separate process in manufacturing a stent.

Also, provided is a coating jig for stents, having a surface roughness, which can improve a production process of an existing stent without adding a process of applying costly silicon powder, oil, or the like. Cost competitiveness can be enhanced through the forgoing by reducing a production cost of a stent.

Additionally, provided is a stent that can be effectively restored to its original shape during a stent procedure even after the stent is stored for a long period of time in a catheter or the like.

The invention is defined in the appended claims.

### BRIEF DESCRIPTION OF THE DRAWINGS

FIG. 1 is a perspective view of a variable coating jig for stents, having a surface roughness, according to an embodiment of the present invention;
FIG 2 is an exploded perspective view of FIG. 1;
FIG. 3 is an example view showing various combinations with respect to a first block portion and a second block portion;
FIG. 4 is a view illustrating that a position of a second block portion continuously changes;
FIG. 5 shows a view of forming a membrane portion by the coating jig of FIG. 1; and
FIG. 6 is a picture which shows an inner face of the membrane portion formed by the coating jig of FIG. 1.

### DETAILED DESCRIPTION OF ILLUSTRATIVE EMBODIMENTS

Example embodiments of the present disclosure are described with reference to the appended drawings to enable a sufficient understanding about elements and effects of the present disclosure. However, the present disclosure is not limited to the disclosed embodiments below. Various forms may be obtained, and various modifications can be applied. Below, in describing the present invention, explanation on related known functions may be omitted if it is determined that the known functions are well-known to one having ordinary skill in the art and may obscure essence of the present invention.

Terms, such as "first," "second," etc., may be used herein to describe various elements. However, the elements should not be understood as being limited by these terms. These terms may be only used in distinguishing one element from another. For example, a first element may be referred to as a second element, and, similarly, a second element may be referred to as a first element, within the scope of the present disclosure.

Herein, terms, such as "comprise," "include," "have," etc., are designed to indicate features, numbers, steps, operations, elements, components, or a combination thereof are present. It should be understood that presence of one or more other features, numbers, steps, operations, elements, components, or a combination thereof or a possibility of addition thereof are not excluded.

Terms used herein are only to explain certain embodiments but not to limit the present invention. A singular representation may include a plural representation unless a clearly different meaning can be grasped from the context. Unless defined differently, terms used in embodiments of the present disclosure may be interpreted as generally known terms to one having ordinary skill in the art.

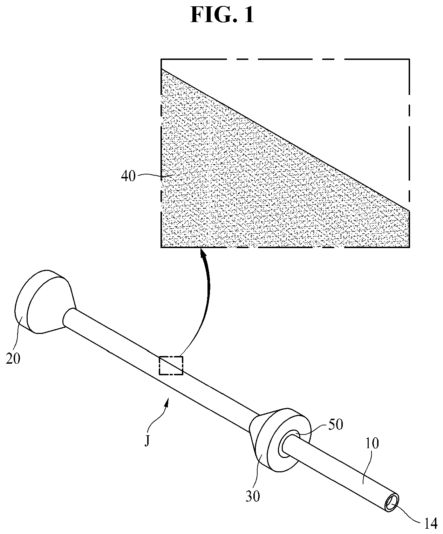

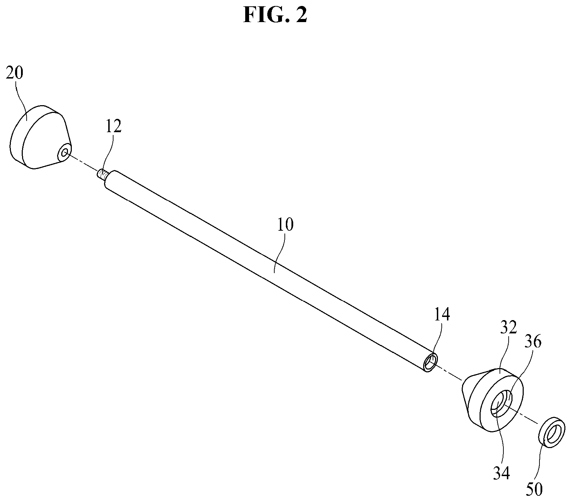

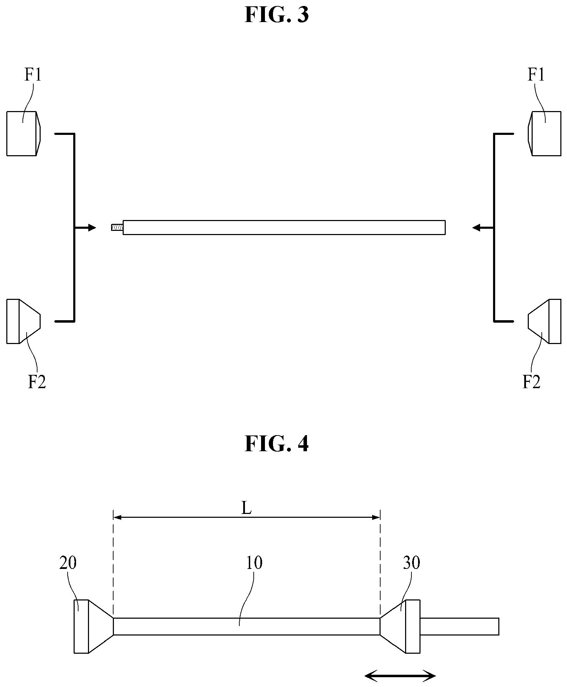

Example embodiments of the present invention are described in detail with reference to the appended drawings. FIG. 1 is a perspective view of a variable coating jig for stents, having a surface roughness, and FIG. 2 is an exploded perspective view of FIG. 1. FIG. 3 is an example view showing various combinations with respect to a first block portion and a second block portion, and FIG. 4 is a view illustrating that a position of a second block portion continuously changes. With reference to FIGS. 1 to 4, a variable coating jig J for stents, having a surface roughness, according to an embodiment of the present invention comprises a jig shaft portion 10, a first block portion 20, a second block portion 30, a surface region 40, a stopper portion 50, and the like.

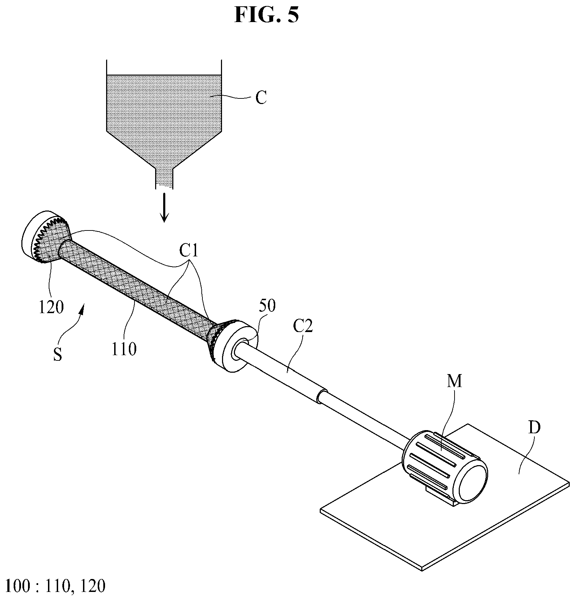

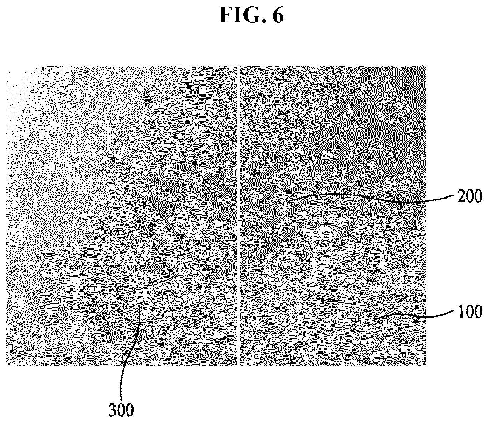

A stent S includes i) a body portion 100 where wires are woven in a zigzag form like a net to form a mesh structure and ii) a membrane portion 200 formed as a thin membrane in empty spaces, i.e., holes that are present between the wires by the mesh structure. The body portion 100 includes a cylindrical tube portion 110 which has a hollow cylindrical shape and an enlarged tubular portion 120 formed at one end of or each end of the cylindrical tube portion 110. The coating jig J according to an embodiment allows a micro-rough surface 300 to be simultaneously formed in the membrane portion 200 while the membrane portion 200 is formed in the body portion 100, as shown in FIGS. 5 and 6.

The jig shaft portion 10 may be circular-shaped, and a cross section of the jig shaft portion 10 may have a predetermined radius r1. The jig shaft portion 10 may be a pivoted element having a circular rod shape that extends longitudinally. The jig shaft portion 10 may be connected to a rotation drive device D disposed outside the coating jig J to be axially rotated. Also, a first sub-coupling portion 12 is arranged at a first end of the jig shaft portion 10. The first sub-coupling portion 12 protrudes in an axial direction of the jig shaft portion 10 and includes a male thread formed on an outer circumference the first sub-coupling portion 12. Corresponding to the first sub-coupling portion 12, a second sub-coupling portion having a shape of a female thread groove is formed at a leading end of the first block portion 20. In addition, a second coupling portion 14 having a groove shape is formed at a second end of the jig shaft portion 10 such that the jig shaft portion 10 may be detachably coupled to the rotation drive device D.

The first block portion 20 is fixedly coupled to the first end of the jig shaft portion 10. To be illustrated, the first block portion 20 may be thread-coupled to the first block portion 20, which fixes a position of the first block portion 20. On the other hand, the second block portion 30 positioned facing the first block portion 20 is variably positioned as sliding along an outer circumference of the jig shaft portion 10. According to an embodiment, the first block portion 20 and the second block portion 30 may be shaped as one of a flange shape F1 and a flare shape F2. Here, the number of combinations of the first block portion 20 and the second block portion 30 is a total of four. On the other hand, design of the first block portion 20 and the second block portion 30 may be changed to any one of shapes of various rotating bodies.

The flange shape F1 may denote a cylindrical shape having a predetermined radius r2. In an embodiment, r2 is greater than r1. The flare shape F2 may indicate a truncated cone shape. In an embodiment, each of a radius r3 of a bottom face and a radius r4 of a top face in the flare shape F2 is greater than r1.

The second block portion 30 includes a rotating body portion 32 and an annular groove portion 36. The rotating body portion 32 includes a penetration hole 34 in a direction of a rotating axis thereof such that both ends of the rotating body portion 32 communicate with each other. The annular groove portion 36 is coaxial with the penetration hole 34 and provided in a lower side of the rotating body portion 32. A minute gap is provided between the outer circumference of the jig shaft portion 10 and an inner circumference formed in the second block portion 30 by the penetration hole 34. When the second block portion 30 is inserted into the jig shaft portion 10 through the penetration hole 34 from the second end of the jig shaft portion 10, the second block portion 30 is slidingly movable in a longitudinal direction of the jig shaft portion 10.

The second block portion 30 according to an embodiment is fixed at a preset position of the jig shaft portion 10. A distance L between the first block portion 20 and the second block portion 30 may be continuously variable. As described above, the second block portion 30 is disposed to face the first block portion 20. In case where the first block portion 20 and/or the second block portion 30 have the flare shape F2, it is preferable that the first block portion 20 and/or the second block portion 30 are coupled to the jig shaft portion 10 in a direction where the top face of the flare shape F2 is first inserted into any one end of the jig shaft portion 10.

In addition, the coating jig J according to an embodiment further comprises the stopper portion 50 which fixes a position of the second block portion 30. However, an arranged position of the stopper portion 50 may be limited to any one region. According to an embodiment, the jig shaft portion 10, the first block portion 20, and the second block portion 30 in the coating jig J include a coated region C1 where a coating material C is applied. The coating material C may be a liquid material used when the membrane portion 200 is formed in the body portion 100. The coated region C1 may include a contact area in which the body portion 100 of the coating jig J makes contact therewith when the body portion 100 is mounted on the coating jig J.

The coated region C1 may comprise an entire area or a part of a side face of the first block portion 20. The coated region C1 may comprise an entire area or a part of a side surface of the second block portion 30. The coated region C1 may comprise all of the outer circumference of the jig shaft portion 10 in a side face of the jig shaft portion 10, in particular, disposed between the first block portion 20 and the second block portion 30. Accordingly, a non-coated region C2 let alone the coated region C1 may be formed in the coating jig J. The non-coated region C2 refers to a region where the coating material C is not applied in a coating process.

The stopper portion 50 according to an embodiment may be disposed in the non-coated region C2, not in the coated region C1. The stopper portion 50 may be formed of a material with a high coefficient of friction, such as synthetic resin and rubber. The stopper portion 50 may be received or disposed in the non-coated region, particularly in the groove portion 36 of the second block portion 30. Here, the stopper portion 50 may have an O-ring shape. The stopper portion 50 is press-fitted into a space formed between the jig shaft portion 10 and the groove portion 36 to fix the position of the second block portion 30.

The variable coating jig J for stents according to an embodiment of the present invention may be widely used in stents having different shapes and sizes. In addition, high process costs required in a conventional coating process can be reduced and installation spaces designated for a coating jig may be minimized.

According to an embodiment, the coating jig J may further comprise the surface region 40 where a surface roughness is formed on at least one or more of the jig shaft portion 10, the first block portion 20, and the second block portion 30. The surface region 40 is formed by irregular depressed grooves which are disorderly arranged. The irregular depressed grooves possess random shapes and forms. The surface region 40 according to an embodiment is not formed by a certain pattern having repetitive and regular characteristics.

The surface region 40 may be formed on an outer face of the coating jig J, i.e., the entire outer face of the coating jig J. For instance, the surface region 40 may be formed on the jig shaft portion 10 in a state where each of the first block portion 20 and the second block portion 30 is assembled and coupled thereto. Also, the surface region 40 may be formed on the entire outer faces of the jig shaft portion 10, the first block portion 20, and the second block portion 30 in a state where the jig shaft portion 10, the first block portion 20, and the second block portion 30 are separate.

A degree of the surface roughness of the surface region 40 may be identified based on a numerical value. Specifically, the surface roughness in the coating jig J may be preferably in a range of tens to hundreds of micrometers (µm) in size. The surface roughness in the coating jig J is in a range of 30 to 300µm. The surface roughness may be changed depending on a component of the coating material C, a viscosity of the coating material C, or the like.

After the coating process where the membrane portion 200 is formed in the coating jig J is finished, the stent S may be collected from the coating jig J. When the surface roughness of the surface region 40 is greater than 300µm, collecting of the stent S where the stent S is separated from the coating jig J becomes difficult. This is because the stent S is not easily separated or detached from the surface of the coating jig J due to the micro-rough surface 300 formed on an inner face of the membrane portion 200. Additionally, if the surface roughness of the surface region 40 is less than 30µm, the existing problem appears due to a stickiness phenomenon where the inner face of the membrane portion 200 sticks to itself.

The coating jig J (or the surface of the coating jig) may be formed of synthetic resin, e.g., polymer material. On the other hand, the coating jig J (or the surface of the coating jig) may be formed of metal. Here, the jig shaft portion 10, the first block portion 20, and the second block portion 30 may be formed of a same material. When the coating jig J is made of the polymer material, the coating jig J (or in a state where the jig shaft portion, the first block portion and the second block portion are separate) may be provided with the surface roughness by a sand blasting process or an abrasive blasting process. In general, the sand blasting process is a process where fine particles of various materials are sprayed with compressed air to clean a surface. However, in an embodiment of the present invention, the sand blasting process is performed to form the surface roughness on the coating jig J.

In the sand blasting process, conditions, such as a spray material, a spray pressure, a spray time or the like, may be changed based on a material of the coating jig J, a thickness of the coating jig J, and the like. For example, the spray material may be hydroxy apatite (HA) or zirconia and glass bid, etc. The sand blasting process may be performed more than at least once to minimize a discrepancy of the surface roughness.

When the coating jig J are made of metal, the coating jig J (or when the coating jig is in a state where the jig shaft portion, the first block portion and the second block portion are separated) may be provided with the surface roughness by an etching process. The coating jig J may be surface-treated through the etching process consisting of several operations. The etching process may further comprise a plurality of pre-treatment operations and a plurality of post-treatment operations. The etching process according to an embodiment may include acid etching where an acid solution is used for etching. To this end, an etching solution may be prepared in an etching reservoir. Subsequently, the coating jig J may be soaked in the etching reservoir under predetermined conditions of temperature and time to etch the surface thereof. On the other hand, the etching solution, for example, may be sprayed onto the surface of the coating jig J.

As a result, the coating jig J is provided with the surface roughness in a consistent range of 30 to 300µm in the surface region 40. Also, mechanical or chemical surface processing may be used to form the surface roughness. The mechanical processing may be microknurling.

When the second end of the jig shaft portion 10 is coupled to the rotation drive device D, the coating jig J may be axially rotated. The rotation drive device D may include at least one or more electric motors M to provide a drive force. When the body portion 100 is mounted on the coating jig J, the coating jig J axially rotates and the polymer material may be evenly applied to the body portion 100.

FIG. 5 shows a view of forming the membrane portion by the coating jig of FIG. 1. Referring to FIG. 5, the rotation drive device D may be controlled by predetermined rotation speed, rotation period, and the like. To initiate the coating process, the body portion 100 is mounted to the coating jig J. When the coating jig J rotates, the coating material C made of the polymer material is applied to the body portion 100. The coating material C may be contained or stored in a receiving portion of a hopper, etc. The coating material C may be sprayed through a nozzle in a liquid state or sprayed in an aerosol state. The coating material C may be generally spread onto the body portion 100 by a centrifugal force caused by the axial rotation of the coating jig J. This allows the coating material C to be uniformly applied to the body portion 100.

The stent S according to an embodiment of the present invention may be manufactured by a coating process using the variable coating jig J for stents, having a surface roughness. In the coating process, the coating material C is exposed to a predetermined temperature atmosphere. The coating material C is cured as a certain time passes. Consequently, the membrane portion 200 is formed in the body portion 100. The coating process according to an embodiment includes a spinning operation of the coating jig J.

In the coating process, the micro-rough surface 300 formed on the inner face of the membrane portion 200 may decrease stickiness of the membrane portion 200. The micro-rough surface 300 is generated while the membrane portion 200 is cured. In other words, when the coating material C is applied to the coating jig J mounted with the body portion 100 in an aerosol state or a liquid state, then the coating jig J spins at a preset rotation speed, etc., to form the coating material C applied to the coating jig J into the membrane portion 200 in a solid state, whereby the micro-rough surface 300 is formed together with the membrane portion 200.

The coating jig J for stents, having a surface roughness, according to an embodiment of the present invention may decrease the stickiness of the membrane portion 200 without adding a separate process in the existing process where the stent S is made.

FIG. 6 is a picture which shows the inner face of the membrane portion formed by the coating jig of FIG. 1. With reference to FIG. 6, the stent S provided with the membrane portion 200 includes the body portion 100, the membrane portion 200, and the micro-rough surface 300. Herein, it is preferable that the membrane portion 200 is resistant to deformation and highly elastic such as to return to its original state when an external force is removed. Moreover, it is preferable that the membrane portion 200 has a smooth surface and a material characteristic of an extremely low coefficient of friction.

The micro-rough surface 300 may be formed on all or at least a part of the inner face of the membrane portion 200 to correspond to the surface region 40. In other words, the micro-rough surface 300 according to an embodiment is not formed on an outer face of the membrane portion 200. In addition, a first region having the micro-rough surface 300 and a second region not having the micro-rough surface 300 may be repeatedly provided in an inner circumference of the membrane portion 200.

The micro-rough surface 300 may be generated through a plurality of irregular embossing protrusions that are disorderly provided. Also, the micro-rough surface 300 may be generated by a plurality of irregular bumps, a plurality of irregular bosses, a plurality of irregular patterns, and the like. In other words, it is excluded that the micro-rough surface 300 according to an embodiment is generated by a pattern having repetitive and regular characteristics.

The surface roughness of the micro-rough surface 300 according to an embodiment may be in a range of tens to hundreds of micrometers. According to an embodiment, it is preferable that the surface roughness of the micro-rough surface 300 according to an embodiment is in a range of 30 to 300µm. As a result, the micro-rough surface 300 according to an embodiment of the present invention may reduce the stickiness of the inner face of the membrane portion 200.

The micro-rough surface 300 may decrease surface contact between the inner faces of the membrane portion 200 when the stent S is received in an insertion instrument. As a result, the micro-rough surface 300 may effectively block increase of the stickiness as the area of the surface contacting with an inner space of the body portion 100 increases. Also, even when the stent S according to an embodiment of the present invention is stored for a long period of time in a catheter, etc., the stent S can be effectively restored into its original shape by the decrease of stickiness of the membrane portion 200.

## Claims

1. A variable coating jig for stents, having a surface roughness, comprising:
a jig shaft portion (10);
a first block portion (20) fixedly coupled to a first end of the jig shaft portion (10);
a second block portion (30) opposingly disposed with respect to the first block portion (20) and variably positioned to slide along an outer circumference of the jig shaft portion (10); and
a surface region (40), wherein a surface roughness is formed on outer surfaces of the jig shaft portion (10), the first block portion (20), and the second block portion (30),
wherein the surface region (40) is formed by irregular depressed grooves that are disorderly formed, rather than by any single type of pattern having a regular characteristic,
wherein the surface roughness is within a range of 30 µm to 300 µm,
wherein the second block portion (30) includes:
a rotating body portion (32) including a penetration hole (34) formed in a direction of a rotating axis of the second block portion (30) such that both ends of the second block portion (30) communicate with each other; and
an annular groove portion (36) coaxially disposed with the penetration hole (34) and provided in a lower side of the rotating body portion (32),
wherein a minute gap is formed between the outer circumferential surface of the jig shaft portion (10) and the penetration hole (34),
wherein the variable coating jig further comprises a stopper portion (50) received in the annular groove portion (36) and fixing a position of the second block portion (30),
wherein the stopper portion (50) is press-fitted into a space formed between the jig shaft portion (10) and the groove portion (36).

2. The variable coating jig of claim 1, wherein the second block portion is fixed at a predetermined position in the jig shaft portion (10), wherein a distance (L) between the first block portion (20) and the second block portion (30) is continuously variable.

3. The variable coating jig of claim 1, wherein a coating region (C1) to which a coating material (C) is applied is provided in the jig shaft portion (10), the first block portion (20), and the second block portion (30).

4. The variable coating jig of claim 1, wherein the surface roughness is generated through a sand blasting process or an etching process.

5. A stent, wherein a membrane portion (200) is formed through a coating process using the variable coating jig for stents according to claim 1.

6. The stent of claim 5, wherein a micro-rough surface (300) is formed on an inner face of the membrane portion (200) such that a stickiness of the membrane portion (200) is decreased.

## Patentansprüche

1. Variable Beschichtungsvorrichtung für Stents mit einer Oberflächenrauheit, umfassend:
einen Vorrichtungsschaftabschnitt (10);
einen ersten Blockabschnitt (20), der fest mit einem ersten Ende des Vorrichtungsschaftabschnitts (10) gekoppelt ist;
einen zweiten Blockabschnitt (30), der in Bezug auf den ersten Blockabschnitt (20) gegenüberliegend angeordnet ist und variabel positioniert ist, um entlang eines Außenumfangs des Vorrichtungsschaftabschnitts (10) zu gleiten; und
einen Oberflächenbereich (40), wobei eine Oberflächenrauheit auf Außenflächen des Vorrichtungsschaftabschnitts (10), des ersten Blockabschnitts (20) und des zweiten Blockabschnitts (30) ausgebildet ist,
wobei der Oberflächenbereich (40) durch unregelmäßige vertiefte Nuten gebildet ist, die ungeordnet ausgebildet sind, anstatt durch irgendeine einzelne Art von Muster mit einer regelmäßigen Charakteristik,
wobei die Oberflächenrauheit in einem Bereich von 30 µm bis 300 µm liegt,
wobei der zweite Blockabschnitt (30) umfasst:
einen Rotationskörperabschnitt (32), der ein Durchgangsloch (34) enthält, das in einer Richtung einer Rotationsachse des zweiten Blockabschnitts (30) ausgebildet ist, sodass beide Enden des zweiten Blockabschnitts (30) miteinander in Verbindung stehen; und
einen ringförmigen Nutabschnitt (36), der koaxial zum Durchgangsloch (34) angeordnet ist und in einer Unterseite des Rotationskörperabschnitts (32) vorgesehen ist,
wobei ein winziger Spalt zwischen der Außenumfangsfläche des Vorrichtungsschaftabschnitts (10) und dem Durchgangsloch (34) ausgebildet ist,
wobei die variable Beschichtungsvorrichtung ferner einen Anschlagabschnitt (50) umfasst, der in dem ringförmigen Nutabschnitt (36) aufgenommen ist und eine Position des zweiten Blockabschnitts (30) fixiert,
wobei der Anschlagabschnitt (50) in einen Raum eingepresst ist, der zwischen dem Vorrichtungsschaftabschnitt (10) und dem Nutabschnitt (36) gebildet ist.

2. Variable Beschichtungsvorrichtung nach Anspruch 1, wobei der zweite Blockabschnitt an einer vorbestimmten Position in dem Vorrichtungsschaftabschnitt (10) fixiert ist, wobei ein Abstand (L) zwischen dem ersten Blockabschnitt (20) und dem zweiten Blockabschnitt (30) kontinuierlich variabel ist.

3. Variable Beschichtungsvorrichtung nach Anspruch 1, wobei ein Beschichtungsbereich (C1), auf den ein Beschichtungsmaterial (C) aufgebracht ist, in dem Vorrichtungsschaftabschnitt (10), dem ersten Blockabschnitt (20) und dem zweiten Blockabschnitt (30) vorgesehen ist.

4. Variable Beschichtungsvorrichtung nach Anspruch 1, wobei die Oberflächenrauheit durch einen Sandstrahlprozess oder einen Ätzprozess erzeugt wird.

5. Stent, wobei ein Membranabschnitt (200) durch einen Beschichtungsprozess unter Verwendung der variablen Beschichtungsvorrichtung für Stents nach Anspruch 1 gebildet ist.

6. Stent nach Anspruch 5, wobei eine mikroraue Oberfläche (300) auf einer Innenfläche des Membranabschnitts (200) ausgebildet ist, sodass eine Klebrigkeit des Membranabschnitts (200) verringert ist.

## Revendications

1. Dispositif de revêtement variable pour stents, présentant une rugosité de surface, comprenant:
une partie tige de dispositif (10);
une première partie bloc (20) accouplée à demeure à une première extrémité de la partie tige de dispositif (10);
une deuxième partie bloc (30) disposée en regard de la première partie bloc (20) et positionnée de manière variable pour coulisser le long d'une circonférence extérieure de la partie tige de dispositif (10); et
une région de surface (40), dans laquelle une rugosité de surface est formée sur les surfaces extérieures de la partie tige de dispositif (10), de la première partie bloc (20) et de la deuxième partie bloc (30),
dans lequel la région de surface (40) est formée par des rainures irrégulières en creux qui sont formées de manière désordonnée, plutôt que par un quelconque type de motif présentant une caractéristique régulière,
dans lequel la rugosité de surface est comprise dans une plage de 30 µm à 300 µm,
dans lequel la deuxième partie bloc (30) comprend:
une partie corps tournant (32) comprenant un trou de pénétration (34) formé dans une direction d'un axe de rotation de la deuxième partie bloc (30) de telle sorte que les deux extrémités de la deuxième partie bloc (30) communiquent entre elles; et
une partie rainure annulaire (36) disposée coaxialement au trou de pénétration (34) et disposée dans une partie inférieure de la partie corps tournant (32),
dans lequel un espace infime est formé entre la surface circonférentielle extérieure de la partie tige de dispositif (10) et le trou de pénétration (34),
dans lequel le dispositif de revêtement variable comprend en outre une partie butée (50) logée dans la partie rainure annulaire (36) et bloquant une position de la deuxième partie bloc (30),
dans lequel la partie butée (50) est emmanchée à force dans un espace formé entre la partie tige de dispositif (10) et la partie rainure (36).

2. Dispositif de revêtement variable selon la revendication 1, dans lequel la deuxième partie bloc est bloquée dans une position prédéterminée dans la partie tige de dispositif (10), dans lequel une distance (L) entre la première partie bloc (20) et la deuxième partie bloc (30) est variable en continu.

3. Dispositif de revêtement variable selon la revendication 1, dans lequel une zone de revêtement (C1) sur laquelle est appliqué un matériau de revêtement (C) se trouve dans la partie tige de dispositif (10), la première partie bloc (20) et la deuxième partie bloc (30).

4. Dispositif de revêtement variable selon la revendication 1, dans lequel la rugosité de surface est obtenue par sablage ou gravure.

5. Stent, dans lequel une partie membrane (200) est formée selon un processus de revêtement faisant appel au dispositif de revêtement pour stents selon la revendication 1.

6. Stent selon la revendication 5, dans lequel une surface micro-rugueuse (300) est formée sur une face interne de la partie membrane (200) de manière à réduire l'adhésivité de la partie membrane (200).
